# EUROPEAN PATENT APPLICATION

(11) **EP 1 671 659 A1**
(43) Date of publication of application: **21.06.2006**
(21) Application number: 04762220.4
(22) Date of filing: 23.09.2004
(51) Int. Cl.: A61L 9/22

(54) **AN AIR PURIFYING APPARATUS**

(30) Priority: 07.10.2003 CN 200310100932
(71) Applicant: Chen, Jinxing, Fuzhou, Fujian 350004 (CN); Wei, Kemei, Fuzhou, Fujian 350002 (CN); Chen, Tao, Fuzhou, Fujian 350002 (CN)
(72) Inventor: CHEN, Jinxing, Fuzhou, Fujian 350004 (CN); WEI, Kemei, Fuzhou, Fujian 350002 (CN); CHEN, Tao, Fuzhou, Fujian 350002 (CN); CHEN, Duanhui, Fuzhou, Fujian 350002 (CN)
(74) Representative: Johnstone, Helen Margaret
(86) International application number: PCT/CN2004/001087
(87) International publication number: WO 2005/037335

(57) **Abstract**

The present invention relates to an air purifying apparatus that includes a casing, an orientation air deflector disposed on the casing, a movable air deflector included in the casing and disposed at a position corresponding to the orientation air deflector, a plasma reactor disposed at the bottom of the movable air deflector, an electric function controller having an anion circuit and a plasma circuit therein and disposed at the bottom of the plasma reactor, a fan box having a fan installed therein and disposed at the bottom of the electric function controller, and a power switch disposed at the bottom of the casing. The air purifying apparatus can achieve the effects of sterilizing, disinfecting, and deodorizing air.

## Description

### BACKGROUND OF THE INVENTION

### (a) Field of the Invention:

The present invention relates to an environmental protective home electric appliance product, and more specifically to a plasma nano catalytic disinfecting and purifying apparatus that integrates the technologies of pulse high-voltage plasma, anion transmitter, and nano catalysis.

### (b) Description of the Related Art:

In the environmental protective products, an air purifier becomes a popular appliance and has a tremendous market potential. The present air purifiers include anion air clarifiers, ozone air purifiers, ultraviolet air purifiers, photocatalytic air purifiers, and plasma air purifiers, and products that integrate different kinds of purifiers. In the comparison of the working efficiency and functions of the aforementioned purifiers, ozone and photocatalytic air purifiers are the most superior ones, but there still exists the shortcomings of a complicated structure and a low performance.

The inventor of the present invention has participated in the researches of the subjects as disclosed in P.R.C. Pat. No. 96217032.2 entitled "Indoor disinfecting purifier", P.R.C. Pat. No. 96217998.1 entitled "Ozone photocatalytic air purifier", P.R.C. Pat. No. 9821668.2 entitled "High-performance ozone water purifier, and Pat. No. JL99209701.C, etc. These patents and the products related to these patented technologies are the applications of low-power plasma and anion technologies.

The patent search for patents including the aforementioned PRC patents indicates that there is no purifier having a high disinfecting and purifying capability and a fast disinfecting rate that integrates plasma and nano catalytic technologies in the market.

### SUMMARY OF THE INVENTION

In view of the shortcomings of the prior art plasma products including the low power, complicated structure and poor disinfecting capability, the inventor of the present invention based on years of experience in the related industry to conduct extensive researches and experiments, and finally invented a plasma nano catalytic disinfecting and purifying apparatus in accordance with the invention.

Therefore, it is a primary objective of the present invention to provide a plasma nano catalytic disinfecting and purifying apparatus to overcome the shortcomings of the prior art.

The measure taken by the present invention to overcome the foregoing shortcomings is to provide a plasma nano catalytic disinfecting and purifying apparatus characterized in that: the apparatus comprises a casing; an orientation air deflector disposed on the casing; a movable air deflector included in the casing and disposed at a position corresponding to the orientation air deflector; a plasma reactor installed below the movable air deflector, and plasma reactor installs an anion anode plate, an anion cathode plate, a plasma anode plate and a plasma cathode plate sequentially from top to bottom, and the anion anode plate, anion cathode plate, plasma anode plate and plasma cathode plate are meshed stainless steel plates, and a thin film of nano catalyst is coated on the surface of the meshed stainless steel plate, and the plasma anode and cathode plates are comprised of 2 to 30 groups, and the nano material is a three-element (copper, titanium and platinum) catalyst; an electric function controller installed at the bottom of the plasma reactor, a fan box having a fan installed therein and disposed below the electric function controller, and the electric function controller contains an anion circuit and a plasma circuit; a power switch disposed at the bottom of the casing; a front panel installed at the lower front side of the casing and at the bottom of the orientation air deflector; a function display device installed at the upper front side of the front panel; and a filter disposed at the lower front side of the front panel. The aforementioned components constitute the plasma nano catalytic disinfecting and purifying apparatus in accordance of the present invention.

The anion and plasma circuits in the electric function controller comprise the following components:
a power circuit, which is a 200V power circuit passing through two fuses F₁, F₂ for its operations, one being connected to the power of a fan, and the speed modulation and remote control circuit of a fan are common fan remote control circuits; a high power plasma and anode transmitting circuit including a power transformer B₁, a bridge rectifier D₁~D₄, a filter circuit C₁, a bleeder resistor R₁, and a dropping resistor R₂, and the high-voltage 220V current passes through the transformer for current transformation, filter, and step-down voltage and then the current is inputted into a three-terminal voltage regulator of IC₁ for voltage regulation and served as a current voltage input VCC of the IC₂ and IC₃;
a pulse oscillation circuit, which is a circuit comprising three nonconjunction gates in IC₂ and W₁, R₃, R₄, W₂, and C₃, and the waveform of the pulse oscillation circuit is a pulse wave whose width is adjusted by a small W₁ and whose frequency curve is adjusted by W₂, and the outputted oscillated pulse wave passes through a fourth non-conjunction gate to activate six NOT gates;
a pulse buffer distribution circuit, forming a circuit with every three NOT gates 6 as a group for the six NOT gates, and the pulse buffer level increases the outputted current, and the frequency and voltage of the pulse oscillation circuit will not be affected when the high power tube load is changed;
a signal input and protect circuit, including a potential rectifier W1, W4 for rectifying the magnitude of pulse signals, an isolating capacitor C₄, C₅, and a high power tube input protection circuit D7, D8; and
a high power field effect power amplifier, including a high power field effect tube GB₁, GB₂, a field effect tube high-voltage protection circuit D₅, D₆, a pulse high-voltage transformer B₂, B₃, and a pulse negative-voltage output D₉ uses as an anion transmitter, a pulse positive-voltage output D₁₀ used as a plasma transmitter, an anion transmitter P1, a plasma transmitter P2, and a nano catalyst coated on the electrode plates of two kinds of transmitters.

After the purifier of the present invention is assembled, the purifier can be put at a predetermined place for disinfecting and purifying indoor air.

The beneficial effects of the present invention include its extensive applications in different areas including hospitals, offices, food industry, computer room, public places and families, etc. For disinfection and sterilization, and prevention of cross infection, the microorganism retaining rate is up to 95% (for two hours). The price of a fan filter unit having a similar effect as the air purifying apparatus of the invention is several times of the air purifying apparatus of the present invention, and the effect of purifying aerosol particles of microorganisms falls in the range of 0.1µm~0.5µm. If the aerosol particle size is smaller than 0.1µm, the high-performance filter installed in the fan filter unit no longer functions properly. The filter of the fan filter unit creates a large resistance to the air, and thus affecting the efficiency of the air purification. In addition, it is necessary to replace the filter regularly. As we know that the size of microorganism's aerosol particles is approximately 0.002µm ~30µm, and the size of bacteria is approximately 0.25µm~20µm, and the size of viruses is approximately 0.002µm~0.30µm, and thus the fan filter unit has a good capability for effectively filtering bacteria, but it is unable to filter viruses. The present invention can collect tiny particles at the level of 0.01 µm~0.001 µm, and also has a more powerful disinfecting strength than that of the fan filter units. The invention concurrently has the functions of purifying various different organic matters and ammonium toxic gases. If different toxic gases are filled into a space of 22.5 m³, the concentration of each gas in equilibrium is given as follows: 4.14 mg/m³ for formaldehyde, 9.37 mg/m³ for benzene, 8.09 mg/m³ for toluene, 5.04 mg/m³ for xylene, and 6.65 mg/ m³ for ammonium, and the purifying rate for each gas after 180 minutes of purification is given as follows: 76.6% for formaldehyde, 87.8% for benzene, 91.8% for toluene, 93.6% for xylene, and 92.9% for ammonium. Thus, the present invention can achieved the expected objectives.

### BRIEF DESCRIPTION OF THE DRAWINGS

The present invention will now be described in more detail hereinafter with reference to the accompanying drawings that show various embodiments of the invention, in which:

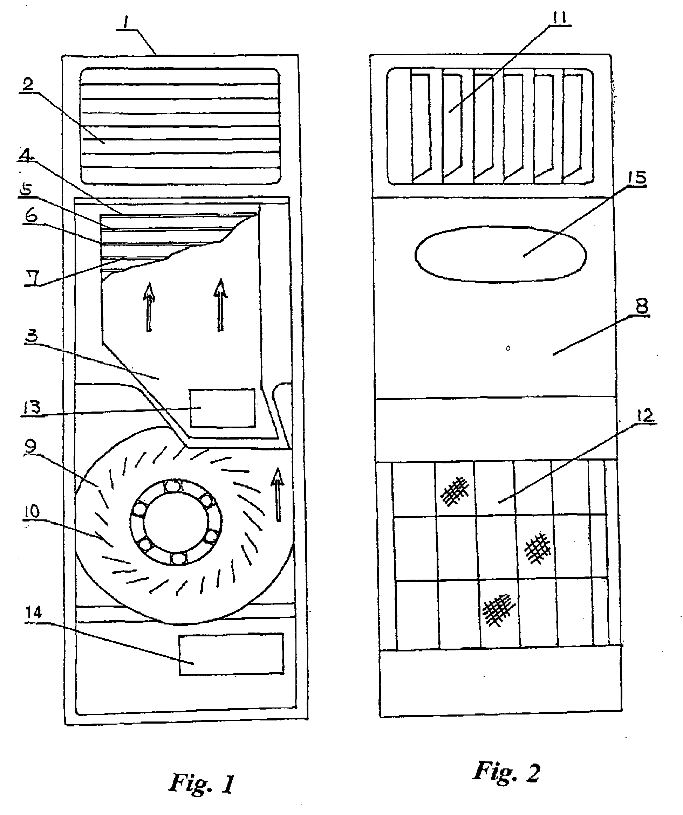

FIG. 1 is a cross-sectional view of a partial structure of a casing of the present invention;
FIG. 2 is a schematic cross-sectional view of another partial structure of a casing of the present invention; and

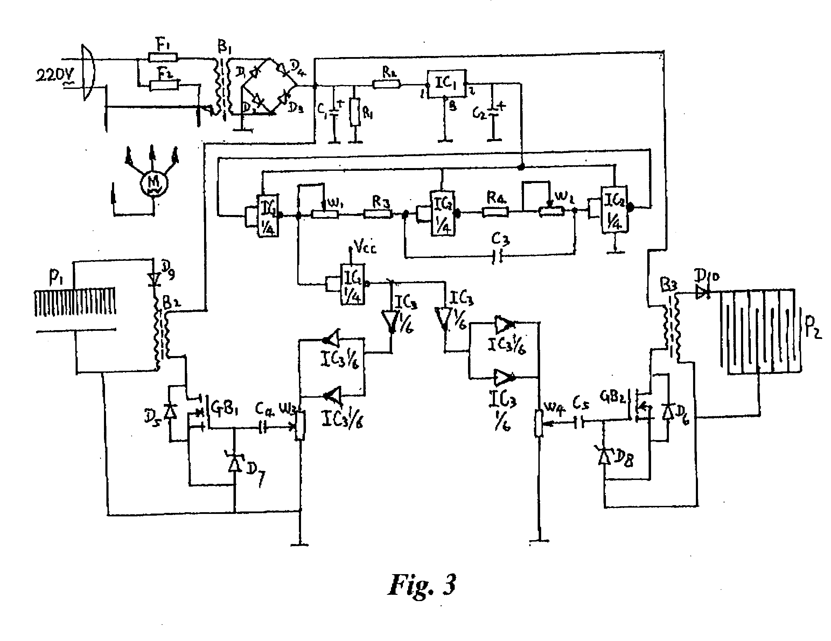

FIG. 3 is a circuit diagram of a high power plasma and anode transmitters of the present invention.

### DETAILED DESCRIPTION OF THE PREFERRED EMBODIMENTS

An air purifying apparatus in accordance with a preferred embodiment of the present invention comprises: a casing 1; an orientation air deflector 2 disposed at the upper portion of the casing 1; a movable air deflector 11 included in the casing 1 and disposed at a position corresponding to the orientation air deflector 2; a plasma reactor 3 installed below the movable air deflector 11, and the plasma reactor 3 installs an anion anode plate 4, an anion cathode plate 5, a plasma anode plate 6 and a plasma cathode plate 7 sequentially from top to bottom, and the anion anode plate 4, anion cathode plate 5, plasma anode plate 6 and plasma cathode plate 7 are meshed stainless steel plates, and a thin film of nano catalyst is coated on the surface of the meshed stainless steel plate, and the plasma anode and cathode plates are comprised of 10 groups, and the nano material is a three-element (copper, titanium and platinum) catalyst; an electric function controller 13 installed at the bottom the plasma reactor 3, a fan box 9 having a fan installed 10 therein and disposed below the electric function controller 13, and the electric function controller 13 contains an anion circuit and a plasma circuit; a power switch 14 disposed at the bottom the casing 1; a front panel 8 installed at a lower front side of the casing 1 and disposed at the bottom of the orientation air deflector 2; a function display device 15 installed at the upper front side of the front panel 8; and a filter 12 disposed at the lower front side of the front panel 8. The aforementioned components constitute the plasma nano catalytic disinfecting and purifying apparatus in accordance of the present invention.

The anion and plasma circuits in the electric function controller comprise the following components:
a power circuit, which is a 200V power circuit passing through two fuses F₁, F₂ for its operations, one being connected to the power of a fan, and the speed modulation and remote control circuit of a fan are common fan remote control circuits; a high power plasma and anode transmitting circuit including a power transformer B₁, a bridge rectifier D₁~D₄, a filter circuit C₁, a bleeder resistor R₁, and a dropping resistor R₂, and the high-voltage 220V current passes through the transformer for current transformation, filter, and step-down voltage and then the current is inputted into a three-terminal voltage regulator of IC₁ for voltage regulation and served as a current voltage input VCC of the IC₂ and IC₃;
a pulse oscillation circuit, which is a circuit comprising three nonconjunction gates in IC₂ and W₁, R₃, R₄, W₂, and C₃, and the waveform of the pulse oscillation circuit is a pulse wave whose width is adjusted by a small W₁ and whose frequency curve is adjusted by W₂, and the outputted oscillated pulse wave passes through a fourth non-conjunction gate to activate six NOT gates;
a pulse buffer distribution circuit, forming a circuit with every three NOT gates 6 as a group for the six NOT gates, and the pulse buffer level increases the outputted current, and the frequency and voltage of the pulse oscillation circuit will not be affected when the high power tube load is changed;
a signal input and protect circuit, including a potential rectifier W1, W4 for rectifying the magnitude of pulse signals, an isolating capacitor C₄, C₅, and a high power tube input protection circuit D7, D8; and
a high power field effect power amplifier, including a high power field effect tube GB₁, GB₂, a field effect tube high-voltage protection circuit D₅, D₆, a pulse high-voltage transformer B₂, B₃, and a pulse negative-voltage output D₉ uses as an anion transmitter, a pulse positive-voltage output D₁₀ used as a plasma transmitter, an anion transmitter P1, a plasma transmitter P2, and a nano catalyst coated on the electrode plates of two kinds of transmitters.

After the purifier of the present invention is assembled, the purifier can be put at a predetermined place for disinfecting and purifying indoor air.

While the invention has been described by way of examples and in terms of preferred embodiments, it is to be understood that the invention is not limited thereto. To the contrary, it is intended to cover various modifications and similar arrangements and procedures, and the scope of the appended claims therefore should be accorded the broadest interpretation so as to encompass all such modifications and similar arrangements and procedures.

## Claims

1. A plasma nano catalytic disinfecting and purifying apparatus, comprising:
a casing;
an orientation air deflector, disposed on said casing;
a movable air deflector, included in said casing and disposed at a position
corresponding to said orientation air deflector;
a plasma reactor, installed below said movable air deflector, and said plasma reactor installs an anion anode plate, an anion cathode plate, a plasma anode plate and a plasma cathode plate sequentially from top to bottom, and said anion anode plate, anion cathode plate, plasma anode plate and plasma cathode plate are meshed stainless steel plates, and a thin film of nano catalyst is coated on the
surface of said meshed stainless steel plate;
an electric function controller, installed at the bottom of said plasma reactor;
a fan box having a fan installed therein and disposed below said electric function controller, and said electric function controller contains an anion circuit and a plasma circuit;
a power switch, disposed at the bottom of said casing;
a front panel, installed at the front side of said casing and disposed at the bottom
of said orientation air deflector;
a function display device, installed at the upper front side of the front panel; and
a filter, disposed at the lower front side of said front panel.

2. The plasma nano catalytic disinfecting and purifying apparatus of claim 1, wherein said plasma anode and cathode plates are comprised of 2~30 groups.

3. The plasma nano catalytic disinfecting and purifying apparatus of claim 1, wherein said nano catalyst is made of a nano material containing three elements of copper, titanium, and ammonium.

4. The plasma nano catalytic disinfecting and purifying apparatus of claim 1, wherein said anion and plasma circuits in said electric function controller comprise:
a power circuit, which is a 200V power circuit passing through two fuses F₁; F₂ for its operations, one being connected to the power of a fan, and the speed modulation and remote control circuit of a fan are common fan remote control circuits; a high power plasma and anode transmitting circuit including a power transformer B₁, a bridge rectifier D₁~D₄, a filter circuit C₁, a bleeder resistor R₁, and a dropping resistor R₂, and the high-voltage 220V current passes through the transformer for current transformation, filter, and step-down voltage and then the current is inputted into a three-terminal voltage regulator of IC₁ for voltage regulation and served as a current voltage input VCC of the IC₂ and IC₃;
a pulse oscillation circuit, which is a circuit comprising three nonconjunction gates in IC₂ and W₁, R₃, R₄, W₂, and C₃, and the waveform of the pulse oscillation circuit is a pulse wave whose width is adjusted by a small W₁ and whose frequency curve is adjusted by W₂, and the outputted oscillated pulse wave passes through a fourth non-conjunction gate to activate six NOT gates;
a pulse buffer distribution circuit, forming a circuit with every three NOT gates as a group for said six NOT gates, and the pulse buffer level increases the outputted current, and the frequency and voltage of said pulse oscillation circuit will not be affected when a high power tube load is changed;
a signal input and protect circuit, including a potential rectifier W1, W4 for rectifying the magnitude of pulse signals, an isolating capacitor C₄, C₅, and a high power tube input protection circuit D7, D8; and
a high power field effect power amplifier, including a high power field effect tube GB₁, GB₂, a field effect tube high-voltage protection circuit D₅, D₆, a pulse high-voltage transformer B₂, B₃, and a pulse negative-voltage output D₉ uses as an anion transmitter, a pulse positive-voltage output D₁₀ used as a plasma transmitter, an anion transmitter P1, a plasma transmitter P2, and a nano catalyst coated on the electrode plates of two kinds of transmitters.
